# EUROPEAN PATENT SPECIFICATION

(11) **EP 3 472 694 B1**
(45) Date of publication and mention of the grant of the patent: **29.07.2026**
(21) Application number: 17813909.3
(22) Date of filing: 13.06.2017
(51) Int. Cl.: G06F 3/048, G06F 15/16, G06F 21/00, G06F 21/62, G06Q 30/018, H04L 9/40, G16H 10/40, G16H 40/20, G16H 40/40

(54) **PROTECTED DATA TRANSFER COORDINATED WITH SERVICE REQUEST**
MIT EINER DIENSTANFRAGE KOORDINIERTE GESCHÜTZTE DATENÜBERTRAGUNG
TRANSFERT DE DONNÉES PROTÉGÉES COORDONNÉ AVEC UNE DEMANDE DE SERVICE

(30) Priority: 17.06.2016 US 201662351433 P
(43) Date of publication of application: 24.04.2019
(73) Proprietor: Siemens Healthcare Diagnostics Inc., Tarrytown, NY 10591 (US)
(72) Inventor: MAGOWAN, Steve, Elkton Maryland 21921 (US); TUDOSE, Catalin, Yorktown Heights New York 10598 (US)
(74) Representative: Schweitzer, Klaus
(86) International application number: PCT/US2017/037187
(87) International publication number: WO 2017/218493

(56) References cited:
- WO-A1-2015/023443
- US-A1- 2003 061 071
- US-A1- 2004 181 368
- US-A1- 2011 185 035
- US-A1- 2011 313 810
- US-A1- 2013 138 452
- US-A1- 2016 058 996
- US-B1- 6 377 162
- US-B1- 6 377 162

## Description

### RELATED APPLICATIONS

This patent application claims priority from United States Provisional Patent Application No. 62/351,433, filed June 17, 2016.

### BACKGROUND

Various embodiments relate generally to systems, methods, devices, and computer programs for the protected transfer of data in conjunction with an instrument service request and, more specifically, relate to encrypting Private Health Information (PHI) or other sensitive information, stored in conjunction with an instrument, before conveying the information to a service provider in conjunction with a service request issued by an instrument user.

This section is intended to provide a background or context. The description may include concepts that may be pursued, but have not necessarily been previously conceived or pursued. Unless indicated otherwise, what is described in this section is not deemed prior art to the description and claims and is not admitted to be prior art by inclusion in this section.

Normally, when medical lab personnel require assistance with respect to a laboratory instrument, a phone call is placed with or an electronic message is sent to a service representative responsible for the instrument. Typically, the service representative requires the laboratory instrument user to provide certain information relating to the configuration of the instrument, including operating system or other software version, physical location, configuration, customer contact, and error messages or other problems associated with the instrument. This can be time consuming and can lead to missing or erroneous data, either through the fault of the user, the service representative, or both. In addition, certain information stored in conjunction with the laboratory instrument should be available only to authorized local personnel and not to the service representative. For example, sensitive or legally protected data, such as patient-related data known as Protected Health Information (PHI), should not be communicated beyond the local facility and should be accessible only by authorized personnel.

US 6,377,162 B1 pertains to a field service unit for providing interactive field service for medical diagnostic systems. The field service unit includes a service platform installed on a portable system, such as a portable computer. The field service unit is adapted to formulate service requests, transmit and receive service data, and render field service to a variety of medical diagnostic system modalities. The field service unit includes communications modules for linking the field service unit to a remote service facility and to subscribing medical diagnostic systems. Service requests may originate in the field service unit, or in the remote service facility, or in the medical diagnostic system, with the field service unit being informed of the state of service requests via a network link.

What is needed is a way to convey required data from a laboratory instrument to a service provider in conjunction with a service request in a secured, accurate, and efficient manner, without exposing protected data to unauthorized access.

### BRIEF SUMMARY

The invention is defined by the independent claim. The below summary is merely representative and non-limiting,

The above problems are overcome, and other advantages may be realized, by the use of the embodiments.

In a first aspect, an embodiment provides a computer-implemented method for gathering and conveying data characteristic of a laboratory instrument to a remote service provider in conjunction with a service request initiated by a user of the laboratory instrument. Certain protected data stored in conjunction with the laboratory instrument is encrypted to avoid unauthorized access by the remote service provider. The laboratory instrument includes one or more analyzers and sample handling equipment. A computer network associated with laboratory instrument includes one or more computer associated with the one or more analyzers and the sample handling equipment, each computer including a respective processor and memory. The one or more computers are configured to communicate via a communications bus that includes one or more data switches. The computer network further includes a router implementing a firewall, the router enabling selective communication between the laboratory instrument and a laboratory or site network. The laboratory or site network enables selective communication with a remote service provider such as via the Internet. The service provider includes a human or virtual, computer-generated representative and a computer system, along with plural services providing service request processing and data transfer and remote collaboration facilitation. The method includes a user initiating a service request with remote service personnel via a service request user interface displayed within a graphical user interface (GUI) associated with the instrument. In the latter case, the GUI may be presented on a display screen integral with the instrument or on a handheld device such as a tablet computer in wireless communication with the instrument. A processor in the computer network is programmed to respond to the service request by gathering predefined data, receiving data entered by the user, encrypting the data, and conveying the data to the service provider via the router and laboratory or site network. The service provider receives the conveyed data, decrypts the data that was encrypted by the laboratory instrument, and provides derivative data to the representative for use in responding to the requesting user. The remote service assigns a ticket identifier to the request and returns this identifier to the requesting laboratory instrument. Additional data, identified by the ticket identifier, is then assembled and transmitted to the remote service. Meanwhile, the requesting user and service provider communicate such as through a voice channel, text message, email, VOIP, or other mechanism, which may include an exchange of voice or data via the same or different communications network used for conveying the conveyed data. The representative provides input to the user at least in part based upon the conveyed data and the derivative data. The remote service may also provide data to the laboratory instrument, such as software updates.

In another aspect, a service request user interface, or service button, is provided on a user interface for a laboratory or medical instrument, such as an in vitro diagnostics instrument. A non-limiting example of an in vitro diagnostics instrument is a system that is capable of performing immunoassay-based and/or clinical chemistry-based tests on biological samples. The user interface is provided on an interactive display unit, such as a touchscreen monitor that is wirelessly connected, for example, an operator tablet computer, via radio frequency transmission, cellular network, or wireless local area network, or physically attached to the laboratory or medical instrument. When the customer actuates the service button feature by interacting with the user interface, relevant system software files containing instrument location, software version, configuration, customer contact, and event codes corresponding to instrument performance and/or status are automatically transmitted to remote support personnel prior to initial contact with the requesting user through an online, voice, and/or video remote support service.

In a further aspect, an embodiment provides an apparatus (such as a server or plural servers) each having a processor and a memory storing computer program code. The memory and the computer program code are configured to, with the respective processor, cause the apparatus to perform the methods above.

In another aspect, an embodiment provides a computer readable medium tangibly encoded with a computer program executable by a processor to perform any of the methods above.

### BRIEF DESCRIPTION OF THE SEVERAL VIEWS OF THE DRAWINGS

Aspects of the described embodiments are more evident in the following description, when read in conjunction with the attached Figures.
Fig. 1 is a schematic diagram of a system for enabling the transfer of protected data in coordination with a service request;
Fig. 2 is a flowchart of the transfer of protected data in coordination with a service request; and
Fig. 3 is a schematic diagram of customer service provider facilities interfacing to a user instrument within the system of Fig. 1.

### DETAILED DESCRIPTION

Various embodiments allow data to be transferred automatically and safely to a service provider in conjunction with a request for assistance initiated by a user of a laboratory instrument. The data may be used by the service provider to assess the operational status of the laboratory instrument prior to or simultaneously with the user interacting with a representative of the service provider. The service provider may then provide information to the requesting user for remediating an instrument problem, explain options for configuring or operating the instrument, or assist with properly configuring the instrument software or hardware environment. In addition, the service provider may initiate a secure transfer of software program code and/or data to the laboratory instrument through the same communications channel by which the data from the laboratory instrument was received in order to update software being executed by the instrument or to request additional data from the instrument for diagnostic purposes.

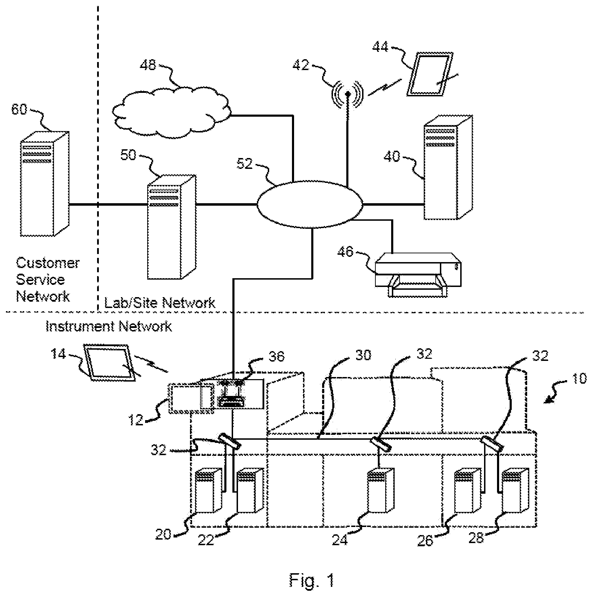

With respect to Fig. 1, a service request interface is provided in association with a laboratory instrument 10. The service request interface can be implemented through a variety of interactive elements. Non-limiting examples include a virtual pushbutton displayed within a graphical user interface (GUI) on a display screen 12 associated with the laboratory instrument or on a display screen of a portable computing device 14 such as a laptop or tablet computer in wireless communication with the laboratory instrument. A specific example of a suitable tablet computer is the Microsoft Surface Pro 4. The wireless communication may be via radio frequency transmission, cellular communications, wireless local area network (WLAN), or Bluetooth-type personal area network (PAN). The display screen may be touch-sensitive or may be provided with one or more soft keys adjacent the screen. The service request interface of the laboratory instrument may include one or more of a speaker and microphone, a telephone handset, a webcam, a keyboard, and a mouse or trackball for enabling interactive communication with personnel of a remote service provider. Certain software functionality implemented within the laboratory instrument may also contribute to the service request interface, including virtual network communications (VNC) or remote desktop capability.

The laboratory instrument may be one of a variety of instruments. Non-limiting examples include a medical laboratory instrument such as an *in vitro* diagnostics instrument capable of performing immuno-based and/or clinical chemistry-based tests on biological samples. The laboratory instrument is typically disposed within a laboratory setting along with other similar or disparate instruments.

The laboratory instrument may comprise one or more computers. For example, in Fig. 1, the depicted instrument 10 includes a sample handling (SH) computer 20, a process control computer (PCC) 22, a chemistry (CH) analyzer 24, an immunoassay (IM) analyzer computer 26, and an immunoassay device management layer (DML) computer 28. The various computers are interconnected via a communications bus 30 and plural switches 32 serving as an interface to the communications bus, and may be provided by, for example, members of the Intel Core or Texas Instruments SITARA^{®} families of processors. Each computer preferably contains security measures for creating a secure platform, such as employing a secured base image (SBI) and implementing "kiosk mode" operations to limit user privileges. A specific embodiment of such an instrument is known as the ATELLICA^{®} Solution of Siemens Medical Solutions USA, Inc., a multi-component system for *in vitro* diagnostic testing of clinical specimens. The system is intended for the qualitative and quantitative analysis of various body fluids, using photometric, turbidimetric, chemiluminescent, and integrated ion selective electrode technology for clinical use. However, other laboratory instruments may also be used with the presently disclosed methods and systems.

Communications from the laboratory instrument to other equipment in the laboratory environment and beyond is preferably through a single access point that implements various security measures. For example, as illustrated, the laboratory instrument of Fig. 1 communicates to the lab or site network via a router 36, such as a Cisco Systems model RV130W. Such a router may limit access to select ports, create private network segments and perform address translation to enable external communications, and enable encryption of data transmitted from the laboratory instrument. Non-limiting examples of encryption techniques that may be implemented by the router include internet protocol security (IPSec), HTTP over SSL (HTTPS), an SSL tunnel, or an HTTP tunnel. The router may also enable local, wireless communications with properly configured devices such as tablet computers 14.

The data stored within the laboratory instrument in association with various constituent computers may include patient-specific protected health information (PHI). Such information is typically required to be encrypted in order to avoid inadvertent disclosure to the public or unauthorized users. The laboratory instrument will typically encrypt PHI stored therein. Ideally, the only person or persons having the virtual key required to decrypt the PHI data are those lab personnel having the proper authority; the remote service provider ideally does not have access to the decryption key or to PHI from the laboratory instrument. Exceptions to the latter include copies of instrument internal databases or database backups that may be transmitted to the remote service provider. However, any PHI found within those database copies is encrypted and not viewable by remote service personnel.

The laboratory instrument communicates with a data network associated with the laboratory or physical site in which it is located. Such a network is referred to as a "Lab/Site Network" in Fig. 1. Typical elements of such a network include a laboratory information system (LIS) computer 40 for managing workflow and data tracking within the respective laboratory, a wireless access point (WAP) 42, one or more wireless tablets or other computers 44, standard network peripherals such as printers 46, all interconnected via a router 52. Remote access is enabled through a connection to the Internet 48 and to a variety of Web-based resources, as known in the art.

Also shown in the laboratory or site network of Fig. 1 is a remote service gateway computer 50 for managing and controlling laboratory instruments registered with the remote service provider, here represented by a remote customer service computer 60, though it is understood that there may be a plurality of such remote service computers, each interfacing with one or more remote laboratory or site networks. Communications of data from the laboratory instrument to the remote service is routed through the remote service gateway computer. Such communications from the remote service gateway computer to the remote customer service computer may be via wireless networks, optical fiber networks, wired networks, etc. As noted above, such communications are encrypted, such as through the use of an SSL tunnel.

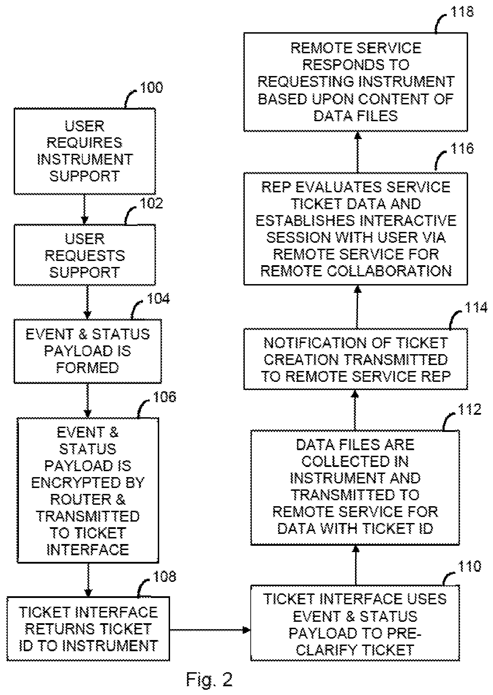

With reference to Fig. 2, a method for an instrument user accessing a remote service according to the present disclosure is illustrated. First, a requesting user, who is typically a lab technician or manager, identifies 100 a condition or problem that requires the assistance of a remote service provider. In response to this identification, the user initiates a support request 102, such as through interaction with a service request user interface (UI), also referred to as a service button UI, provided on a display associated with the laboratory instrument or portable computing device 14 communicating with the instrument. The UI provides multiple functions. First, it enables a user to request an interactive communications session with remote service personnel. This may be via telephone, speaker and microphone associated with the laboratory instrument, text message, email, videoconference, or other means. Second, the service button UI enables a user to review the status of the most recent service request, also referred to as a service ticket, and the status of previously initiated service tickets. Service requests that have been resolved are designated "closed" while those still in process are designated "open." Third, the service button UI enables a user with flexibility as to the data that will be provided to a remote service in association with the service ticket request. This will be discussed in greater detail, below.

In response to entry of a service request by the user via the service button UI, the process control computer 22 of the laboratory instrument automatically assembles predefined data sets 104 that may be referred to as an event and status payload or event codes. For example, data identifying the laboratory user requesting the service, their contact information, relevant system software data containing instrument serial number, physical location, software version configuration data, and event codes corresponding to the instrument performance and/or status are accumulated by the process control computer. The assembled data also includes a ticket priority entered by the requesting user. In one embodiment, the priority is selected from: urgent; non-urgent; and general inquiry /no problem. In another embodiment, the service button UI provides the requesting user with two free-edit texts fields: a ticket title field, which may be limited to 40 characters, for example; and a problem description field, which may be limited to 2000 characters, for example. Further, a requesting user is asked to provide a four digit passcode that may be used by a remote service provider to engage the requesting user is a collaborative session to address the service request.

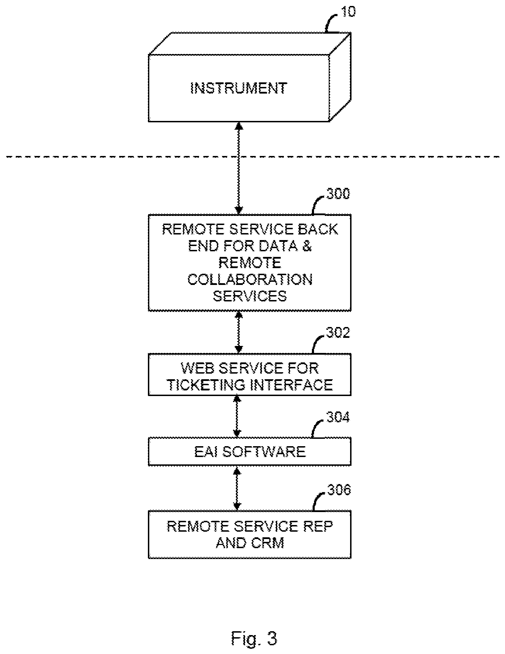

With reference also to Fig. 3, the process control computer 22 of the instrument 10 enters the event and status payload into, for example, a simple object access protocol (SOAP) protocol message and transmits 106 the event and status payload with ticket request to a web service for a ticketing interface 302 via the router 36 and remote service gateway computer 50. All such communications are encrypted and sent via a secure channel such as an SSL tunnel.

At the ticketing interface 302, a new ticket ID is generated and associated with the received, decrypted, and locally stored event and status payload. The ticketing interface returns 108 the ticket ID to the laboratory instrument 10 using the SOAP protocol via the same secure SSL tunnel communications channel. The process control computer 22 enters the new ticket ID into a list of tickets created via the service button UI along with the respective status of the ticket ID. In one embodiment, the process control computer identifies the status of a ticket as "open" unless confirmed "closed" by input from the ticketing interface. At any time, a user may use the service button UI to determine the current status of a previously opened ticket, a process that involves the process control computer querying the ticketing interface for a return message indicative of current status. For each listed ticket, in one embodiment, there is an associated display of creation date and time, most recent status (open versus closed), and the date and time of the most recent status.

The decrypted and locally stored event and status payload data is indexed into a data structure referred to as a service ticket, or more simply, the ticket, associated with the respective ticket ID. Associating the event and status payload data with the respective ticket is referred to as pre-clarifying the ticket 110. The ticket structure subsequently enables remote service personnel to have access to the event and status payload information as fielded in an easily recognizable format.

In addition to the creation of the service ticket, as described in the foregoing, the process control computer 22 identifies a minimum set of predefined data files within the laboratory instrument that are always provided to the remote service whenever a new service ticket is initiated. In one embodiment, the requesting user is also given an opportunity, via the service button UI, to select and send additional data files. The ticket ID returned to the laboratory instrument from the ticketing interface is then used to identify the instrument data files, which are then transmitted 112 to the a remote service back end for data and remote collaboration services 300, via the same secure pathway as the ticket request, event and status payload data, and returned ticket ID. Certain PHI may be included in the with the other instrument data files, such as if a backup of an instrument database is performed. However, the PHI will always be encrypted within the instrument prior to being transmitted to the remote service provider. As noted above, the remote service personnel do not have access to the information necessary to decrypt the PHI.

Once the ticket has been pre-clarified within the ticketing interface 302 and the instrument data files, associated with the same ticket ID as the pre-clarified ticket, have been received and stored with the back end for data services process 300, a notification is sent 114 to remote service personnel 306 responsible for responding to the service request. Such a request, in one embodiment, is transmitted via an enterprise application integration (EAI) software module 304 such as an SAP NETWEAVER^{®} process integration (SAP-PI) module. The service representative now has access to: the service ticket data stored within the ticketing interface 302; the instrument data stored within the back end for data service 300; and a communications channel with the requesting user via the back end for remote collaboration service 300. At this point, the service rep enters into an interactive exchange with the requesting user 116 using the four-digit code defined by the requesting user at the time of initial service request. The rep is able to respond to problems or inquiries while automatically having access to detailed instrument configuration and status information. Thus, when voice, data or other interactive communications are established between the requesting user and the remote service provider, the latter already has the situational context with which to more efficiently and thoroughly assist the requesting user. This avoids the time consuming process of having the requesting user respond to inquiries from the remote service provider for the same information, which would otherwise present the opportunity for transcription errors or miscommunicated information. The remote service provider is then prepared to provide necessary information to the requesting user as soon as a communication link is established.

Depending upon the configuration and operational status of the laboratory instrument, as reflected in the service ticket data and the instrument data, the remote service rep may utilize the same secure data channel to provide data and/or executable files back to the process control computer 22 of the laboratory instrument 10 for the purpose of patching, updating or deleting existing software files, installing new software, or performing other required maintenance to the software or hardware configuration of the laboratory instrument. If all of the issues raised by the requesting user are adequately addressed by the remote service personnel and any optional reconfiguration of instrument software has been carried out by the remote service via the secure channel, the remote service personnel changes the status of the service ticket in the ticketing interface web service 302 to "closed." The next time service ticket status is updated from the instrument service button UI, the status display within that UI will also change to "closed," along with the date and time of the revised status.

Any of the operations described that form part of the presently disclosed embodiments may be useful machine operations. Various embodiments also relate to a device or an apparatus for performing these operations. The apparatus can be specially constructed for the required purpose, or the apparatus can be a general-purpose computer selectively activated or configured by a computer program stored in the computer. In particular, various general-purpose machines employing one or more processors coupled to one or more computer readable media, described below, can be used with computer programs written in accordance with the teachings herein, or it may be more convenient to construct a more specialized apparatus to perform the required operations.

The procedures, processes, and/or modules described herein may be implemented in hardware, software, embodied as a computer-readable medium having program instructions, firmware, or a combination thereof. For example, the functions described herein may be performed by a processor executing program instructions out of a memory or other storage device.

The foregoing description has been directed to particular embodiments. However, other variations and modifications may be made to the described embodiments, with the attainment of some or all of their advantages. It will be further appreciated by those of ordinary skill in the art that modifications to the above-described systems and methods may be made without departing from the concepts disclosed herein. Accordingly, the invention should not be viewed as limited by the disclosed embodiments. Furthermore, various features of the described embodiments may be used without the corresponding use of other features. Thus, this description should be read as merely illustrative of various principles, and not in limitation of the invention.

## Claims

1. A method for initiating customer service via a laboratory instrument (10), the method comprising:
receiving notification of a user request for service via the laboratory instrument (10);
in response to receiving the notification, generating, by a processor within the laboratory instrument (10), service request payload data comprising event codes corresponding to laboratory instrument configuration and status;
encrypting the service request payload data at an interface to the laboratory instrument (10);
transmitting the service request payload data from the laboratory instrument (10) to a ticketing interface remote service (302);
decrypting the service request payload data at the ticketing interface remote service (302); and
receiving communications, by the laboratory instrument processor (22) from the ticketing interface remote service (302), regarding a ticket identifier associated with the service request payload data;
generating, by the laboratory instrument processor (22), instrument data files associated with the ticket identifier; and
transmitting the instrument data files to a data remote service for storage, the instrument data files identifiable within the data remote service by the respective ticket identifier,
transmitting the ticket identifier to a remote representative;
assembling a service reply message, by the remote representative, based upon the instrument data files associated with the ticket identifier in the data remote service; and
transmitting the service reply message to the laboratory instrument processor;
wherein the service reply message comprises instructions to modify software executable by the laboratory instrument processor (22),
the method further comprising indexing the decrypted and locally stored event and status payload data into a data structure referred to as a service ticket, wherein the service ticket is associated with the respective ticket identifier and
the remote service providing data and/or executable files back to the process control computer (22) of the laboratory instrument (10) for the purpose of patching, updating or deleting existing software files, installing new software, or performing other required maintenance to the software configuration of the laboratory instrument based upon the service ticket and the instrument data,
wherein the laboratory instrument (10) encrypts protected health information stored therein.

2. The method of claim 1, wherein the service request payload data further includes at least one of:
a location of the laboratory instrument (10);
software version information of software running on the laboratory instrument (10);
data associated with a patient sample under test within the laboratory instrument (10); and
identity of the user requesting service.

3. The method of claim 1, further comprising accessing software data files associated with the laboratory instrument (10) to determine the service request payload data.

4. The method of claim 1, wherein generating the service request payload data comprises automatically filling out data fields in a service ticket request message corresponding to the service request payload data.

5. The method of claim 1, further comprising generating, by the ticketing interface remote service (302), the ticket identifier upon transmission of the user request for service and the encrypted service request payload data to the ticketing interface remote service (302) and storing the decrypted service request payload data in association with the ticket identifier.

6. The method of claim 1, wherein the instrument data files comprise at least one of: log files, event characterizing data, display screen capture information, database backup files, diagnostic data, and laboratory instrument maintenance records.

7. The method of claim 1, wherein the step of generating instrument data files further comprises enabling a user requesting service to select laboratory instrument data files to be included in the instrument data files to be transmitted to the data remote service.

## Patentansprüche

1. Verfahren zum Initiieren eines Kundendienstes über ein Laborgerät (10), wobei das Verfahren umfasst:
Empfangen einer Benachrichtigung über eine Benutzeranforderung für einen Dienst über das Laborgerät (10);
als Reaktion auf das Empfangen der Benachrichtigung, Erzeugen, durch einen Prozessor innerhalb des Laborgeräts (10), von Nutzdaten einer Dienstanforderung, die Ereigniscodes umfassen, die einer Konfiguration und dem Zustand des Laborgeräts entsprechen;
Verschlüsseln der Nutzdaten der Dienstanforderung an einer Schnittstelle zu dem Laborgerät (10);
Übertragen der Nutzdaten der Dienstanforderung von dem Laborgerät (10) zu einem Ferndienst für eine Ticketschnittstelle (302);
Entschlüsseln der Nutzdaten der Dienstanforderung in dem Ferndienst für eine Ticketschnittstelle (302); und
Empfangen von Kommunikationen, in dem Laborgeräteprozessor (22) von dem Ferndienst für eine Ticketschnittstelle (302) bezüglich einer Ticketkennung, die mit den Nutzdaten der Dienstanforderung assoziiert ist;
Erzeugen, durch den Laborgeräteprozessor (22), von Dateien mit Gerätedaten, die mit der Ticketkennung assoziiert sind; und
Übertragen der Dateien mit Gerätedaten an einen Datenferndienst für eine Speicherung, wobei die Dateien mit Gerätedaten innerhalb des Datenferndienstes durch die entsprechende Ticketkennung identifizierbar sind;
Übertragen der Ticketkennung an einen fernen Repräsentanten;
Zusammenstellen einer Dienstantwortnachricht durch den fernen Repräsentanten basierend auf den Dateien mit Gerätedaten, die in dem Datenferndienst mit der Ticketkennung assoziiert sind; und
Übertragen der Dienstantwortnachricht an den Laborgeräteprozessor;
wobei die Dienstantwortnachricht Anweisungen zum Modifizieren einer Software umfasst, die durch den Laborgeräteprozessor (22) ausführbar ist,
wobei das Verfahren ferner ein Indexieren der entschlüsselten und lokal gespeicherten Ereignis- und Zustandsnutzdaten in eine Datenstruktur umfasst, die als ein Dienstticket bezeichnet wird, wobei das Dienstticket mit der entsprechenden Ticketkennung assoziiert ist, und
wobei der Ferndienst Daten und/oder ausführbare Dateien zurück für den Prozesssteuercomputer (22) des Laborgeräts (10) bereitstellt, mit dem Zweck bestehende Softwaredateien zu patchen, zu aktualisieren oder zu löschen, eine neue Software zu installieren oder andere erforderliche Wartungsarbeiten an der Softwarekonfiguration des Laborgeräts basierend auf dem Serviceticket und den Gerätedaten durchzuführen;
wobei das Laborgerät (10) geschützte Gesundheitsinformationen verschlüsselt, die darin gespeichert sind.

2. Verfahren nach Anspruch 1, wobei die Nutzdaten der Dienstanforderung ferner mindestens eines beinhalten von:
einem Standort des Laborgeräts (10);
Informationen über eine Softwareversion der Software, die in dem Laborgerät ausgeführt wird (10);
Daten, die mit einer zu testenden Patientenprobe innerhalb des Laborgeräts (10) assoziiert sind; und
einer Identität des Benutzers, der den Dienst anfordert.

3. Verfahren nach Anspruch 1, das ferner ein Zugreifen auf Dateien mit Softwaredaten umfasst, die mit dem Laborgerät (10) assoziiert sind, um die Nutzdaten der Dienstanforderung zu ermitteln.

4. Verfahren nach Anspruch 1, wobei das Erzeugen der Nutzdaten der Dienstanforderung ein automatisches Ausfüllen von Datenfeldern in einer Anforderungsnachricht für ein Dienstticket umfasst, das den Nutzdaten der Dienstanforderung entspricht.

5. Verfahren nach Anspruch 1, das ferner ein Erzeugen, durch den Ferndienst für eine Ticketschnittstelle (302), der Ticketkennung nach dem Übertragen der Benutzeranfrage für einen Dienst und der verschlüsselten Nutzdaten der Dienstanforderung an den Ferndienst für eine Ticketschnittstelle (302), und ein Speichern der entschlüsselten Nutzdaten der Dienstanforderung in Verbindung mit der Ticketkennung umfasst.

6. Verfahren nach Anspruch 1, wobei die Dateien mit den Gerätedaten mindestens eine umfassen von: Protokolldateien, Ereignischarakterisierungsdaten, Erfassungsinformationen für einen Anzeigebildschirm, Datenbanksicherungsdateien, Diagnosedaten und Wartungsdatensätzen der Laborgeräte.

7. Verfahren nach Anspruch 1, wobei der Schritt des Erzeugens von Dateien der Gerätedaten ferner ein Ermöglichen umfasst, dass ein Benutzer, der einen Dienst anfordert, Dateien mit Laborgerätedaten auswählt, die in den Dateien der Gerätedaten enthalten sind, die an den Datenferndienst übertragen werden.

## Revendications

1. Procédé de lancement de service client un instrument de via laboratoire (10), le procédé comprenant :
la réception d'une notification d'une demande utilisateur de service via l'instrument de laboratoire (10) ;
en réponse à la réception de la notification, la génération, par un processeur à l'intérieur de l'instrument de laboratoire (10), de données de charge utile de demande de service comprenant des codes d'événement correspondant à une configuration et un état d'instrument de laboratoire ;
le chiffrement des données de charge utile de la demande de service au niveau d'une interface à l'instrument de laboratoire (10) ;
la transmission des données de charge utile de demande de service de l'instrument de laboratoire (10) à un service à distance d'interface d'établissement de ticket (302) ;
le décryptage des données de charge utile de demande de service au niveau du service à distance d'interface d'établissement de ticket (302) ; et
la réception de communications, par le processeur (22) d'instrument de laboratoire en provenance du service à distance d'interface d'établissement de ticket (302), concernant un identifiant de ticket associé aux données de charge utile de demande de service ;
la génération, par le processeur (22) d'instrument de laboratoire, de fichiers de données d'instrument associés à l'identifiant de ticket ; et
la transmission des fichiers de données d'instrument à un service à distance de données pour stockage, les fichiers de données d'instrument étant identifiables au sein du service à distance de données par l'identifiant de ticket respectif,
la transmission de l'identifiant de ticket à un représentant à distance ;
l'assemblage d'un message de réponse de service, par le représentant à distance, sur la base des fichiers de données d'instrument associés à l'identifiant de ticket dans le service à distance de données ; et
la transmission du message de réponse de service au processeur d'instrument de laboratoire ;
le message de réponse de service comprenant des instructions pour modifier un logiciel exécutable par le processeur (22) d'instrument de laboratoire,
le procédé comprenant en outre l'indexation des données de charge utile d'événement et d'état décryptées et stockées localement dans une structure de données appelée ticket de service, le ticket de service étant associé à l'identifiant de ticket respectif et
le service à distance fournissant des données et/ou des fichiers exécutables à l'ordinateur de commande de processus (22) de l'instrument de laboratoire (10) dans le but de corriger, de mettre à jour ou de supprimer des fichiers logiciels existants, d'installer un nouveau logiciel ou d'effectuer une autre maintenance requise de la configuration logicielle de l'instrument de laboratoire sur la base du ticket de service et des données d'instrument,
l'instrument de laboratoire (10) chiffrant des informations de santé protégées stockées dans celui-ci.

2. Procédé selon la revendication 1, les données de charge utile de demande de service comportant en outre au moins un parmi :
un emplacement de l'instrument de laboratoire (10) ;
des informations de version de logiciel fonctionnant sur l'instrument de laboratoire (10) ;
des données associées à un échantillon de patient sous test dans l'instrument de laboratoire (10) ; et
une identité de l'utilisateur demandant un service.

3. Procédé selon la revendication 1, comprenant en outre l'accès à des fichiers de données logicielles associés à l'instrument de laboratoire (10) pour déterminer les données de charge utile de demande de service.

4. Procédé selon la revendication 1, la génération des données de charge utile de demande de service comprenant le remplissage automatique de champs de données dans un message de demande de ticket de service correspondant aux données de charge utile de demande de service.

5. Procédé selon la revendication 1, comprenant en outre la génération, par le service à distance d'interface d'établissement de ticket (302), de l'identifiant de ticket lors de la transmission de la demande utilisateur de service et des données de charge utile de demande de service chiffrées au service à distance d'interface d'établissement de ticket (302) et le stockage des données de charge utile de demande de service décryptées en association avec l'identifiant de ticket.

6. Procédé selon la revendication 1, les fichiers de données d'instrument comprenant au moins un parmi : des fichiers journaux, des données de caractérisation d'événement, des informations de capture d'écran d'affichage, des fichiers de sauvegarde de base de données, des données de diagnostic et des enregistrements de maintenance d'instrument de laboratoire.

7. Procédé selon la revendication 1, l'étape de génération de fichiers de données d'instrument comprenant en outre le fait de permettre à un utilisateur demandant un service de sélectionner des fichiers de données d'instrument de laboratoire à inclure dans les fichiers de données d'instrument à transmettre au service à distance de données.
